# EUROPEAN PATENT APPLICATION

(11) **EP 3 287 776 A1**
(43) Date of publication of application: **28.02.2018**
(21) Application number: 17194842.5
(22) Date of filing: 15.06.2016
(51) Int. Cl.: G01N 27/12, G01N 33/00

(54) **MICRO HEATER AND MICRO SENSOR**

(30) Priority: 18.06.2015 KR 20150086722; 18.06.2015 KR 20150086766; 30.06.2015 KR 20150092688
(62) Divisional of application: 16174642.5
(71) Applicant: Point Engineering Co., Ltd., Asan-si, Chungcheongnam-do 31409 (KR)
(72) Inventor: AHN, Bum Mo, 16509 Suwon-si (KR); PARK, Seung Ho, 18599 Hwaseong-si (KR); BYUN, Sung Hyun, 18452 Hwaseong-si (KR)
(74) Representative: Peter, Julian

(57) **Abstract**

A micro heater includes a substrate and a heater electrode formed on the substrate, wherein a protective layer is formed on the heater electrode.

## Description

### BACKGROUND

### 1. Technical Field

The present invention relates to a micro heater and a micro sensor. More particularly, the present invention pertains to a micro heater and a micro sensor in which multiple pores are formed to vertically extend through a substrate.

### 2. Description of Related Art

As an interest on an environment gradually increases in recent years, a demand has existed for the development of a small-size sensor capable of accurately obtaining different kinds of information within a short period of time. Particularly, for the purpose of making a residential space pleasant, coping with a harmful industrial environment and managing a production process of beverage and foodstuff, efforts have been made to achieve the size reduction, precision enhancement and price reduction of a micro sensor such as a gas sensor for measuring a gas concentration or the like.

The currently available gas sensor gradually evolves from a ceramic-sintered gas sensor or a thick-film-type gas sensor to a micro gas sensor having the form of a micro electro mechanical system (MEMS) due to the application of a semiconductor process technique. From the viewpoint of a measurement method, a method of measuring a change in the electric characteristics of a sensing material of a sensor when a gas is adsorbed to the sensing material is most frequently used in the currently available gas sensor. Typically, a metal oxide such as SnO₂ or the like is used as the sensing material to measure a change in the electrical conductivity depending on the concentration of a measurement target gas. This measurement method has an advantage in that it is relatively easy to use the method. A change in the measurement value becomes conspicuous when the metal oxide sensing material is heated to and operated at a high temperature. Accordingly, accurate temperature control is essential in order to rapidly and accurately measure a gas concentration. Furthermore, the gas concentration is measured after the sensing material is reset or restored to an initial state by forcibly removing gas species or moisture already adsorbed to the sensing material through high-temperature heating. Thus, the temperature characteristics in the gas sensor directly affect major measurement factors such as the sensor measurement sensitivity, the restoration time, the reaction time and the like.

Accordingly, a micro heater configured to locally and uniformly heat only the region of a sensing material is effective for efficient heating. However, if a large amount of electric power is consumed in controlling a temperature when measurement is performed by a micro gas sensor, it is necessary to use a large battery or a large power supply source although the volume of a sensor and a measurement circuit remains small. This may decide the overall size of a measurement system. Thus, in order to realize a micro gas sensor, a structure having small power consumption need to be preferentially taken into account.

Thus far, a silicon substrate having extremely large heat conductivity has been predominantly used in manufacturing most of micro gas sensors. Therefore, in order to reduce a heat loss, an etched pit or groove is formed in a sensor structure through a bulk macro-machining, thereby forming a suspended structure separated from a substrate.

Thereafter, a micro heater, an insulation film and a sensing material are sequentially formed on the suspended structure. This makes it possible to partially reduce a heat transfer loss. However, this method is a manufacturing method primarily focused on wet etching that makes use of the crystal directivity of the substrate. Thus, this method has a limit in reducing the size of a sensor element. Furthermore, there is a problem in that the physical property of an etchant such as KOH (potassium hydroxide) or the like used in this method lacks compatibility with a standard CMOS semiconductor process.

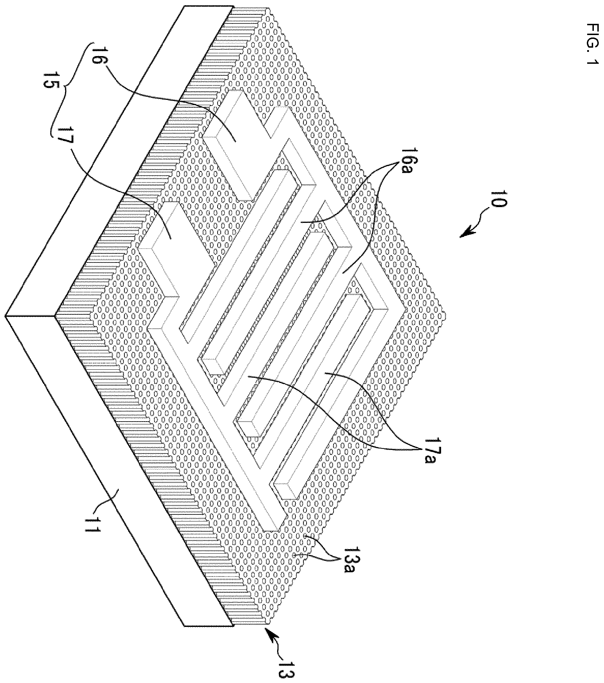

FIG. 1 is a perspective view of a humidity sensor, one of conventional micro sensors. The humidity sensor 10 includes a substrate 11, a porous anodic aluminum oxide (AAO) layer 13 formed on the substrate 11, and an electrode 15 formed on the porous anodic aluminum oxide layer 13.

The substrate 11 is made of aluminum and is formed in a substantially rectangular plate shape. The porous anodic aluminum oxide layer 13 is formed by oxidizing the substrate 11. If aluminum is oxidized, it is possible to form the porous anodic aluminum oxide layer 13 having a plurality of holes 13a formed on the surface thereof. A barrier layer is formed between the porous anodic aluminum oxide layer 13 and the substrate 11.

In this case, the holes 13a are formed to have a diameter of 60 nm or less. By forming the holes 13a to have a diameter of 60 nm or less, it is possible to prevent the holes 13a from being damaged by an etching solution. The electrode 15 is made of metal such as platinum, aluminum, copper or the like. The electrode 15 may be formed by different methods such as a vapor deposition method or the like.

The electrode 15 includes a first electrode 16 and a second electrode 17 disposed adjacent to the first electrode 16. The first electrode 16 has a plurality of electrode projections 16a protruding toward the second electrode 17. The second electrode 17 has a plurality of electrode projections 17a protruding toward the first electrode 16.

However, the micro sensor described above has a problem in that a thermal insulation property is reduced and a heat loss is generated.

In the meantime, when forming electrode patterns, a liquid photoresist is often used in order to secure a pattern resolution. However, if the liquid photoresist is applied to a conventional micro sensor having a porous layer, there is posed a problem in that the liquid photoresist flows into pores and the patterns are not smoothly formed. Another problem is posed in that the electrode is oxidized or damaged.

### [Prior Art Documents]

### [Patent Documents]

Korean Patent Application Publication No. 2009-0064693

Korean Patent No. 1019576

### SUMMARY

In view of the problems mentioned above, it is an object of the present invention to provide a micro heater and a micro sensor, which are capable of improving a heat insulation property and achieving a high temperature through the use of reduced electric power.

According to one aspect of the present invention, there is provided a micro heater, including: a substrate; and a heater electrode formed on the substrate, wherein the substrate includes a plurality of pores formed to vertically extend through the substrate.

The substrate may formed by anodizing an aluminum material and then removing the aluminum material and a barrier layer so that the pores vertically extend through the substrate, and the heater electrode may be formed on a surface of the substrate from which the aluminum material and the barrier layer are removed.

The substrate may be formed by anodizing an aluminum material and then removing the aluminum material and a barrier layer so that the pores vertically extend through the substrate, and the heater electrode may be formed on a surface of the substrate opposite to a surface from which the aluminum material and the barrier layer are removed.

The height of an upper surface of the substrate in a region where the heater electrode is formed may be smaller than the height of the upper surface of the substrate in the remaining region.

According to another aspect of the present invention, there is provided a micro sensor, including: a substrate; a sensor electrode formed on the substrate; and a heater electrode formed on the substrate, wherein the substrate includes a plurality of pores formed to vertically extend through the substrate.

According to a further aspect of the present invention, there is provided a micro heater, including: a substrate; and a heater electrode formed on the substrate, wherein a protective layer is formed on the heater electrode.

The heater electrode may be made of at least one member selected from a group consisting of Pt, W, Co, Ni, Au, Cu and C and a mixture thereof, and the protective layer may be made of at least one member selected from a group consisting of tantalum oxide, titanium oxide, silicon oxide, aluminum oxide, indium tin oxide, indium oxide and tin oxide.

The heater electrode may be made of at least one member selected from a group consisting of Pt, W, Co, Ni, Au, Cu and C and a mixture thereof, an intermediate layer may be disposed between the substrate and the heater electrode, and the intermediate layer may be made of at least one member selected from a group consisting of tantalum oxide, titanium oxide, silicon oxide, aluminum oxide, indium tin oxide, indium oxide and tin oxide.

The substrate may include a plurality of pores formed to vertically extend through the substrate.

The protective layer may be formed in a portion of the heater electrode, and a non-protective portion in which the protective layer is not formed may be formed in the heater electrode.

The heater electrode may include a heater wiring line and heater electrode pads connected to the heater wiring line, and the non-protective portion may be formed on a portion of the heater electrode pads.

The protective layer may be formed on an upper portion and a side portion of the heater electrode.

The protective layer may be formed on the substrate.

According to a still further aspect of the present invention, there is provided a micro sensor, including: a substrate; a sensor electrode formed on the substrate; and a heater electrode formed on the substrate, wherein a protective layer is formed on an upper portion of at least one of the heater electrode and the sensor electrode.

According to a yet still further aspect of the present invention, there is provided a micro heater, including: a substrate including a porous layer having a plurality of vertically-extending pores and a barrier layer positioned on the porous layer; and a heater electrode formed on the barrier layer.

The heater electrode may be formed using a liquid photoresist.

The barrier layer may be partially removed so that the pores of the porous layer vertically extend through the porous layer.

The heater electrode may be made of platinum, and a tantalum oxide layer may be disposed between the substrate and the heater electrode.

According to an even yet still further aspect of the present invention, there is provided a micro sensor, including: a substrate including a porous layer having a plurality of vertically-extending pores and a barrier layer positioned on the porous layer; a sensor electrode formed on the substrate; and a heater electrode formed on the substrate, wherein at least one of the sensor electrode and the heater electrode is formed on the barrier layer.

According to an additional even yet still further aspect of the present invention, there is provided a micro heater, including: a porous substrate having a plurality of pores; and a heater electrode formed on the substrate, wherein the pores disposed under the heater electrode have closed top ends.

The micro heater and the micro sensor of the present invention mentioned above may provide the following effects.

Since the pores are formed in the substrate so as to vertically extend through the substrate, it is possible to improve a heat insulation property and to achieve a high temperature through the use of reduced electric power. Furthermore, since the electrodes are stably supported by the porous layer, it is possible to maintain the mechanical durability. Moreover, it is possible to prevent the electrodes from being damaged by the organic substances remaining within the pores in a heat treatment process. In addition, it is possible to optimally adapt the micro heater and the micro sensor to a small device such as a mobile device or the like.

The substrate is formed of the porous anodic aluminum oxide layer. It is therefore easy to form the porous layer. The heater electrode is formed on the surface of the substrate from which the aluminum material and the barrier layer are removed. In this case, it is possible to form the heater electrode on the surface which is better in planarity than the surface of the porous layer. This makes it possible to enhance the bonding strength of the heater electrode.

The heater electrode may be formed on the surface of the substrate opposite to the surface from which the aluminum material and the barrier layer are removed. In this case, the aluminum material and the barrier layer can be removed after the heater electrode is formed. Thus, when forming the heater electrode or the sensing material, the aluminum material and the barrier layer serve to support the porous layer, thereby maintaining the porous layer.

Since the air gaps are formed so as to surround the heater wiring line, the micro heater has a reduced thermal capacity and can be heated to a high temperature through the use of reduced electric power.

Since the height of the upper surface of the substrate in the region where the heater electrode is formed is smaller than the height of the upper surface of the substrate in the remaining region, it is possible to easily form the heater electrode. Furthermore, the pores are disposed around the heater electrode. An air exists within the pores. Thus, a plurality of air columns is disposed around the heater electrode. Since the air columns serving as heat insulation materials are disposed around the heater electrode in this way, it is possible to prevent the heat of the heater electrode from being transferred to the side surface. This makes it possible to further enhance the heat insulation efficiency of the micro heater. Since the protective layer is formed on the heater electrode, it is possible to prevent the heater electrode from being oxidized. This makes it possible to protect the heater electrode.

The heater electrode is made of at least one member selected from a group consisting of Pt, W, Co, Ni, Au, Cu and C and a mixture thereof. The protective layer is made of at least one member selected from a group consisting of tantalum oxide, titanium oxide, silicon oxide, aluminum oxide, indium tin oxide, indium oxide and tin oxide. It is therefore possible to effectively protect the heater electrode and to effectively prevent oxidation of the heater electrode.

The intermediate layer is disposed between the substrate and the heater electrode. This makes it possible to improve the bonding strength of the heater electrode.

There is provided the non-protective portion in which the protective layer is not formed. The non-protective portion is formed in the region of the sensor wiring lines covered with the sensing material. It is therefore possible for the micro sensor to accurately perform a sensing operation. Furthermore, the non-protective portion is formed in a portion of the heater electrode pads and the sensor electrode pads. It is therefore possible to smoothly perform a soldering work.

The protective layer is formed in the upper portion and the side portion of the heater electrode. The protective layer is also formed on the substrate. It is therefore possible to effectively protect the heater electrode and the substrate.

The heater electrode is formed on the barrier layer of the substrate. Thus, when forming the heater electrode, the liquid photoresist is prevented from flowing into the pores. It is therefore possible to smoothly form the patterns of the heater electrode. It is also possible to accurately form the patterns of the heater electrode in a small size. Due to the existence of the porous layer formed in the substrate, it is possible to improve a heat insulation property and to achieve a high temperature through the use of reduced electric power. Furthermore, since the electrodes are stably supported by the porous layer, it is possible to maintain the mechanical durability. Moreover, it is possible to prevent the electrodes from being damaged by the organic substances remaining within the pores in a heat treatment process. In addition, it is possible to optimally adapt the micro heater and the micro sensor to a small device such as a mobile device or the like. When forming the electrodes or the sensing material, the barrier layer plays a role of supporting the porous layer of the substrate. Thus, the porous layer is maintained.

Since the heater electrode is formed using the liquid photoresist, it is possible to reduce the size of a product and to finely form the electrode patterns. The barrier layer is formed only in a portion of the porous layer. The porous layer includes the pores vertically extending through the porous layer. It is therefore possible to further improve the heat insulation property. The heater electrode is made of platinum. The tantalum oxide layer is disposed between the substrate and the heater electrode. It is therefore possible to increase the bonding strength of the electrode.

### BRIEF DESCRIPTION OF THE DRAWINGS

FIG. 1 is a perspective view showing a conventional humidity sensor.

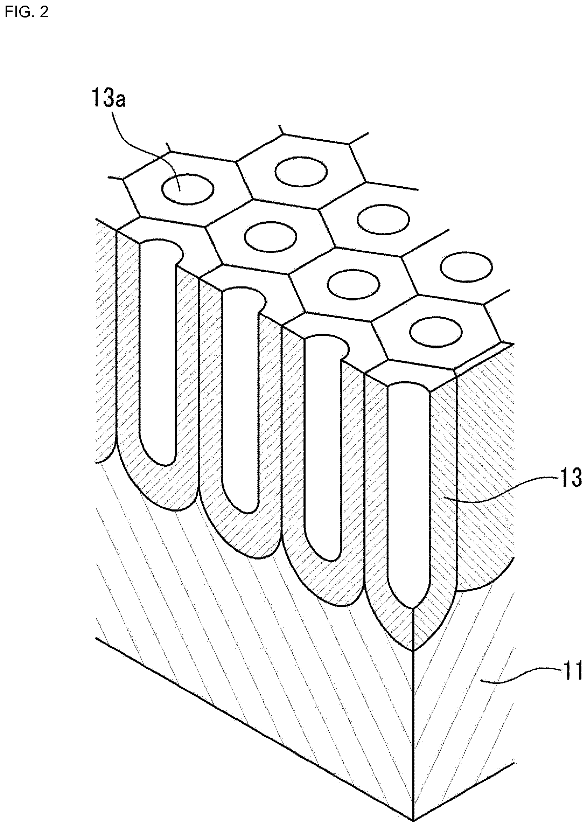

FIG. 2 is a partially-cutaway perspective view showing a conventional porous aluminum oxide layer.
FIG. 3 is a plane view of a micro sensor provided with a micro heater according to a first embodiment of the present invention (in which a sensing material is omitted).
FIG. 4 is a sectional view taken along line A-A in FIG. 3.
FIG. 5 is an enlarged view of the portion indicated by B in FIG. 4.
FIG. 6 is a sectional view of a micro sensor provided with a micro heater according to a second embodiment of the present invention.
FIG. 7 is an enlarged view of the portion indicated by C in FIG. 6.
FIG. 8 is a sectional view of a micro sensor provided with a micro heater according to a third embodiment of the present invention.
FIG. 9 is a sectional view of a micro sensor provided with a micro heater according to a fourth embodiment of the present invention.
FIG. 10 is an enlarged view of the portion indicated by D in FIG. 9.

### DETAILED DESCRIPTION

Preferred embodiments of the present invention will now be described in detail with reference to the accompanying drawings. For reference, in the following descriptions, the same configurations of the present invention as those of the related art will not be described in detail. Reference is made to the foregoing descriptions of the related art.

### <First Embodiment of the Present Invention>

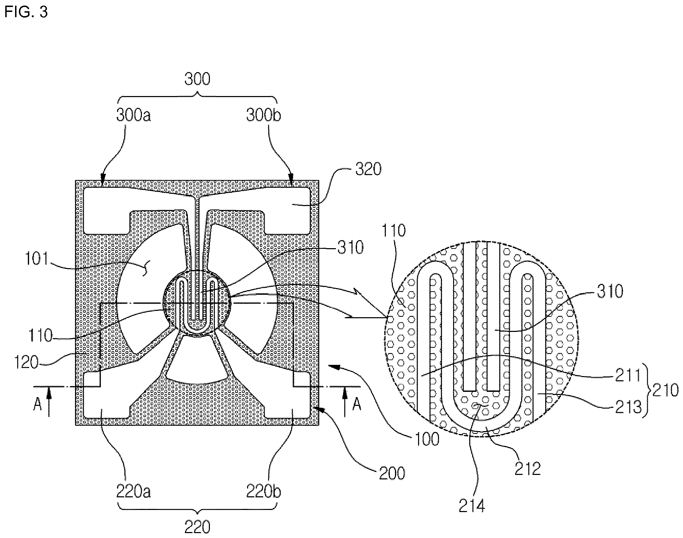

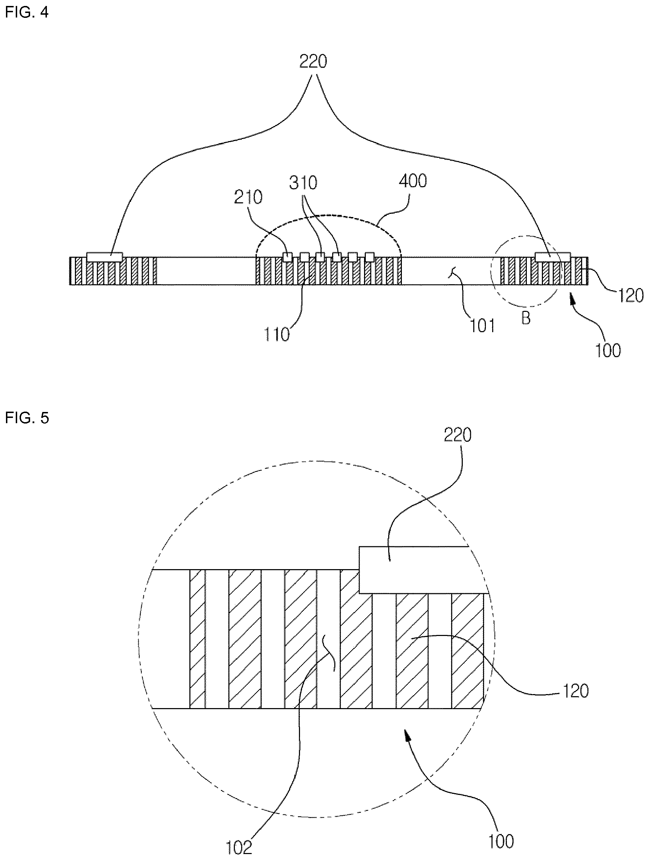

As illustrated in FIGS. 3 to 5, a method for manufacturing a micro sensor provided with a micro heater according to a first embodiment includes a step of forming a substrate 100, and a step of forming a heater electrode 200 and a sensor electrode 300 on the substrate 100.

The substrate 100 as a porous layer having a plurality of vertically-extending pores 102 is formed. For the sake of convenience in description, the diameter and length of the pores 102 are slightly exaggerated in the drawings.

At the step of forming the substrate 100 as a porous layer, a porous anodic aluminum oxide (AAO) layer is formed by anodizing an aluminum material. If the surface (upper surface) of the aluminum material is anodized, pores 102 having open top ends and closed bottom ends are formed. Thus, the aluminum substrate includes pores 102, a barrier layer disposed under the pores 102, and an aluminum material disposed under the barrier layer.

Then, air gaps 101 are formed in the aluminum substrate by etching the aluminum substrate. The regions of the upper surface of the aluminum substrate, in which the heater electrode 200 and the sensor electrode 300 are to be formed, are etched so that the height of the upper surface becomes lower in the etched regions than the remaining regions. The heater electrode 200 and the sensor electrode 300 are formed in the etched regions of the aluminum substrate having a reduced height. The heater electrode 200 and the sensor electrode 300 may be formed by sputtering or the like. The heater electrode 200 and the sensor electrode 300 are formed on the opposite surface of the aluminum substrate from the aluminum material and the barrier layer.

If the porous layer is supported by the aluminum material and the barrier layer when forming the heater electrode 200 and the sensor electrode 300 in this way, it is possible to maintain the porous layer even if the heater electrode 200 and the sensor electrode 300 are formed by sputtering or the like.

The heater electrode 200 includes a heater wiring line 210 and heater electrode pads 220 connected to the heater wiring line 210. The sensor electrode 300 includes sensor wiring lines 310 and sensor electrode pads 320 connected to the sensor wiring lines 310. The heater wiring line 210 and the sensor wiring lines 310 are disposed in the central region of the aluminum substrate. The heater electrode pads 220 and the sensor electrode pads 320 are disposed outside the heater wiring line 210 and the sensor wiring lines 310.

Unlike the foregoing descriptions, the air gaps 101 may be formed after forming the heater electrode 200 and the sensor electrode 300.

The method for manufacturing the micro sensor provided with the micro heater according to the first embodiment further includes a step of forming a sensing material 400 on the upper surface of the aluminum substrate so as to cover the heater wiring line 210 and the sensor wiring lines 310.

The aluminum material existing under the aluminum substrate is removed and then the barrier layer is removed. Thus, the substrate 100 includes only the porous layer.

If the aluminum material and the barrier are removed from the aluminum substrate in this way, the pores 102 are opened at the bottom ends thereof. As a result, the pores 102 having open top and bottom ends are formed in the substrate 100 so as to extend vertically.

As described above, the heater electrode 200 and the sensor electrode 300 are formed on the surface of the aluminum substrate opposite to the surface from which the aluminum material and the barrier layer are removed.

Unlike the foregoing descriptions, the heater electrode 200 and the sensor electrode 300 may be formed on the surface of the aluminum substrate from which the aluminum material and the barrier layer are removed. In this case, it is possible to form the heater electrode 200 and the sensor electrode 300 on the surface which is better in planarity than the surface of the porous layer. This makes it possible to enhance the bonding strength of the heater electrode 200 and the sensor electrode 300.

In this way, the heater electrode 200 and the sensor electrode 300 are formed on the substrate 100 in which the pores 102 having open top and bottom ends are formed. Thus, a vertical air layer is formed below and around the heater electrode 200 and the sensor electrode 300 due to the air existing within the pores 102. Consequently, the micro sensor according to the present embodiment can enjoy an improved heat insulation property and can achieve a high temperature through the use of reduced electric power.

The micro sensor manufactured by the aforementioned micro sensor manufacturing method includes a substrate 100, a heater electrode 200 formed on the substrate 100, and a sensor electrode 300 formed on the substrate 100. A plurality of vertically-extending pores 102 is formed in the substrate 100.

The substrate 100 is made of aluminum and is formed in a substantially rectangular plate shape. The substrate 100 is formed as a porous layer. That is to say, the substrate 100 is made of a porous material. Thus, a plurality of pores 102 having open top and bottom ends is formed in the substrate 100 so as to vertically extend through the substrate 100.

The substrate 100 may be formed by anodizing an aluminum plate. Thus, the porous substrate mentioned above is a porous anodic aluminum oxide (AAO) layer. Since the aluminum material and the barrier layer are removed from the aluminum oxide substrate 100, the pores 102 of the substrate 100 vertically extend through the substrate 100.

The sensor electrode 300 is formed on the upper surface of the substrate 100. The sensor electrode 300 is configured to sense a gas. Unlike the foregoing descriptions, the sensor electrode 300 may be configured to sense humidity or the like. The sensor electrode 300 includes a first sensor electrode 300a and a second sensor electrode 300b disposed in a spaced-apart relationship with the first sensor electrode 300a. The first sensor electrode 300a and the second sensor electrode 300b are spaced apart from each other in a left-right direction and are symmetrical with respect to a centerline vertically extending on a plane.

The first sensor electrode 300a includes a first sensor wiring line and a first sensor electrode pad connected to the first sensor wiring line. The second sensor electrode 300b includes a second sensor wiring line and a second sensor electrode pad connected to the second sensor wiring line.

The sensor wiring lines 310 include the first sensor wiring line and the second sensor wiring line. The sensor electrode pads 320 include the first sensor electrode pad and the second sensor electrode pad.

The sensor wiring lines 310 are disposed in the central region of the substrate 100. The sensor wiring lines 310 are formed in a linear shape with a uniform width. The sensor electrode pads 320 are formed so as to have a width larger than the width of the sensor wiring lines 310. Furthermore, the sensor electrode pads 320 are larger in area than the sensor wiring lines 310 when viewed in a plane view.

The sensor electrode pads 320 of the first and second sensor electrodes 300a and 300b are respectively disposed at two adjoining corners of the substrate 100 having a rectangular shape and are formed so that the width thereof grows larger toward the free ends thereof. In other words, the sensor electrode pads 320 are formed so that the width thereof grows smaller toward the sensor wiring lines 310.

The heater electrode 200 is formed on the upper surface of the substrate 100. Thus, the upper ends of the pores 102 disposed under the heater electrode 200 and the sensor electrode 300 are closed by the heater electrode 200 and the sensor electrode 300. The lower ends of the pores 102 disposed under the heater electrode 200 and the sensor electrode 300 are opened. The upper end and the lower end of at least one of the pores 102 disposed in the region where the heater electrode 200 and the sensor electrode 300 are not formed (the region existing around the heater electrode 200 and the sensor electrode 300) are opened.

In this way, the heater electrode 200 is formed on the porous layer. Due to the existence of the pores 102, a heat insulation effect is enhanced.

Furthermore, the heater electrode 200 and the sensor electrode 300 are formed in the regions of the substrate 100 where the height of the upper surface is smaller than the height of the upper surface of the remaining region. In other words, the heater electrode 200 and the sensor electrode 300 are formed in the depressed regions of the substrate 100. Thus, the thickness of the substrate 100 in the regions where the heater electrode 200 and the sensor electrode 300 are formed is smaller than the thickness of the substrate 100 in the remaining region.

The heater electrode 200 includes a heater wiring line 210 disposed closer to the sensor wiring lines 310 than the sensor electrode pads 320, and heater electrode pads 220 connected to the heater wiring line 210.

The heater wiring line 210 is disposed in the central region of the substrate 100. The heater wiring line 210 includes a first curved portion 211, a second curved portion 213 spaced apart from the first curved portion 211, and a curved connection portion 212 configured to interconnect the first curved portion 211 and the second curved portion 213. When viewed in a plane view, the first curved portion 211 and the second curved portion 213 are curvilinearly formed in an inverted U-like shape. When viewed in a plane view, the curved connection portion 212 is curvilinearly formed in a U-like shape. Thus, an isolated space portion 214 is defined between the first curved portion 211 and the second curved portion 213. The sensor wiring lines 310 are disposed in the isolated space portion 214. In other words, the heater wiring line 210 is formed so as to surround at least some portions of the first sensor electrode 300a and the second sensor electrode 300b. This makes it possible to effectively heat a sensing material 400 which will be described later.

The heater electrode pads 220 include first and second heater electrode pads 220a and 220b respectively connected to the opposite ends of the heater wiring line 210. In this way, there are formed at least two heater electrode pads 220.

The heater electrode pads 220 are disposed at two corners of the substrate 100 other than the corners at which the sensor electrode pads 320 are disposed. The heater electrode pads 220 are formed so that the width thereof grows larger outward. In other words, the heater electrode pads 220 are formed so that the width thereof grows smaller toward the heater wiring line 210. When viewed in a plane view, the heater electrode pads 220 are larger in area than the heater wiring line 210.

A discoloration-preventing protection layer (not shown) is formed over the entirety of the heater electrode 200 and the sensor electrode 300. The discoloration-preventing protection layer may be made of an oxide-based material. Alternatively, the discoloration-preventing protection layer may be made of silicon dioxide or aluminum oxide.

Furthermore, soldering metals are disposed in the end portions of the heater electrode pads 220 and the sensor electrode pads 320. The soldering metals are disposed on the discoloration-preventing protection layer. The soldering metals may be at least one of gold, silver and tin.

Air gaps 101 are formed in the substrate 100 so as to surround the heater wiring line 210 and the sensor wiring lines 310. The air gaps 101 are disposed around the heater wiring line 210 and the sensor wiring lines 310. The maximum width (left-right width) of the air gaps 101 is set larger than the maximum width of the pores 102. In the present embodiment, three air gaps 101 are formed in an arc shape. The air gaps 101 are spaced apart along the circumferential direction. In other words, the air gaps 101 are discontinuously formed in a plural number.

More specifically, the air gaps 101 are disposed between the sensor electrode pad 320 of the first sensor electrode 300a and the first heater electrode pad 220a, between the first heater electrode pad 220a and the second heater electrode pad 220b, and between the second heater electrode pad 220b and the sensor electrode pads 320 of the second sensor electrode 300b. That is to say, the air gaps 101 are formed in the regions other than the regions which support the heater electrode 200 and the sensor electrode 300.

The air gaps 101 vertically extend through the substrate 100. In other words, the air gaps 101 are formed to extend from the upper surface of the substrate 100 to the lower surface thereof. Unlike the foregoing descriptions, the air gaps 101 may be formed in a groove shape.

Due to the existence of the air gaps 101, a first support portion 110 which supports both the heater wiring line 210 and the sensor wiring lines 310 and a second support portion 120 which supports the heater electrode pads 220 and the sensor electrode pads 320 are formed in the substrate 100. That is to say, the air gaps 101 are formed between the first support portion 110 and the second support portion 120. A heat generation peak temperature becomes higher as the width of the air gaps 101 grows wider.

The first support portion 110 is formed in a circular shape so as to support the heater wiring line 210 and the sensor wiring lines 310. The first support portion 110 and the second support portion 120 are connected to each other in the regions where the wiring lines and the pads are connected to each other. In the remaining regions, the first support portion 110 and the second support portion 120 are spaced apart from each other. Accordingly, the first support portion 110 and the second support portion 120 are connected to each other at three points.

The first support portion 110 is formed in a circular shape and is surrounded by the air gaps 101. The first support portion 110 is larger in area than the heater wiring line 210 and the sensor wiring lines 310. The air gaps 101 are formed so as to surround the first support portion 110. Due to the existence of an air exists in the air gaps 101, the heat insulation effect is enhanced, the heat conductivity is reduced, and the thermal capacity is made small.

In addition, a sensing material 400 which covers the heater wiring line 210 and the sensor wiring lines 310 is formed on the first support portion 110. That is to say, the sensing material 400 is formed in the position corresponding to the first support portion 110. The sensing material 400 is formed by printing. If the sensing material 400 is formed by printing in this way, a mesh-like mark is left on the surface of the sensing material 400 after the sensing material 400 is formed.

Next, descriptions will be made on the operation of the micro sensor provided with the micro heater according to the first embodiment of the present invention.

In order to measure a gas concentration, electric power is first applied to the two heater electrode pads 220 of the heater electrode 200, thereby heating the sensing material 400 formed in the central portion of the micro sensor to a predetermined temperature.

In this state, a change in the characteristics of the sensing material 400 generated when a gas existing around the micro sensor is adsorbed to or desorbed from the sensing material 400 is measured by measuring a potential difference between the sensor electrode pads 320 electrically connected to the sensing material 400 via an external circuit and then quantifying the electrical conductivity of the sensing material 400.

In an effort to accurately perform the measurement, gas species or moisture already adsorbed to the sensing material 400 is forcibly removed by heating the same to a high temperature with the heater electrode 200. After the sensing material 400 is restored to an initial state in this way, the concentration of a gas of interest is measured.

### <Second Embodiment of the Present Invention>

Next, descriptions will be made on a micro sensor provided with a micro heater according to a second embodiment of the present invention. In the following descriptions, the components identical with or similar to those of the micro sensor and the micro heater of the first embodiment will be designated by like reference numerals with the detailed description and illustration thereof omitted.

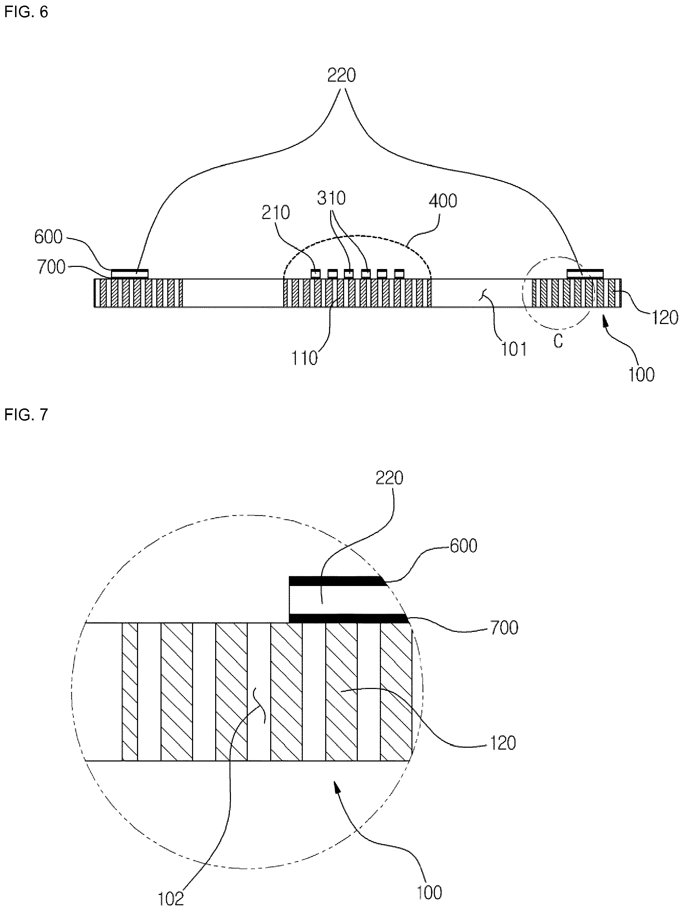

As illustrated in FIGS. 6 and 7, the micro sensor provided with the micro heater according to the second embodiment includes a substrate 100, a sensor electrode formed on the substrate 100, and a heater electrode formed on the substrate 100. A protective layer 600 is formed on at least one of the heater electrode and the sensor electrode

The sensor electrode includes a first sensor electrode, and a second sensor electrode spaced apart from the first sensor electrode. Each of the first and second sensor electrodes includes sensor wiring lines 310 and sensor electrode pads connected to the sensor wiring lines 310. The sensor electrode is formed on the upper surface of the substrate 100 from which the aluminum material and the barrier layer are removed. The sensor electrode may be made of one of Pt, W, Co, Ni, Au, Cu and C or a mixture thereof.

The heater electrode includes a heater wiring line 210 disposed closer to the sensor wiring lines 310 than the sensor electrode pads, and heater electrode pads 220 connected to the heater wiring line 210.

The heater electrode is formed on the upper surface of the substrate 100 from which the aluminum material and the barrier layer are removed. Unlike the foregoing descriptions, the heater electrode and the sensor electrode may be formed on the surface of the substrate 100 opposite to the surface from which the aluminum material and the barrier layer are removed. The heater electrode may be made of one of Pt, W, Co, Ni, Au, Cu and C or a mixture thereof.

A protective layer 600 is formed over the entirety of the heater electrode and the sensor electrode. The protective layer 600 may be made of at least one of tantalum oxide (TaOx), titanium oxide (TiO₂), silicon oxide (SiO₂), aluminum oxide (Al₂O₃), indium tin oxide (ITO), indium oxide (In₂O₃) and tin oxide (SnO₂). The protective layer 600 prevents the heater electrode and the sensor electrode from being oxidized. Thus, the heater electrode and the sensor electrode are protected by the protective layer 600.

Furthermore, an intermediate layer 700 is disposed between the upper surface of the substrate 100 and the lower surfaces of the heater electrode and the sensor electrode. The bonding strength of the heater electrode and the sensor electrode is increased by the intermediate layer 700. The intermediate layer 700 may be made of at least one of tantalum oxide (TaOx), titanium oxide (TiO₂), silicon oxide (SiO₂), aluminum oxide (Al₂O₃), indium tin oxide (ITO), indium oxide (In₂O₃) and tin oxide (SnO₂).

Air gaps 101 are formed in the substrate 100 so as to surround the heater wiring line 210 and the sensor wiring lines 310. Due to the existence of the air gaps 101, a first support portion 110 which supports both the heater wiring line 210 and the sensor wiring lines 310 and a second support portion 120 which supports the heater electrode pads 220 and the sensor electrode pads are formed in the substrate 100. A sensing material 400 is formed so as to cover the upper surfaces and the side surfaces of the heater wiring line 210 and the sensor wiring lines 310 and so as to cover the upper surface of the first support portion 110.

A method of measuring a gas concentration through the use of the micro sensor according to the second embodiment is the same as the method of the first embodiment described above. Thus, the detail descriptions thereof will be omitted.

### <Third Embodiment of the Present Invention>

Next, descriptions will be made on a micro sensor provided with a micro heater according to a third embodiment of the present invention. In the following descriptions, the components identical with or similar to those of the micro sensor and the micro heater of the first and second embodiments will be designated by like reference numerals with the detailed description and illustration thereof omitted.

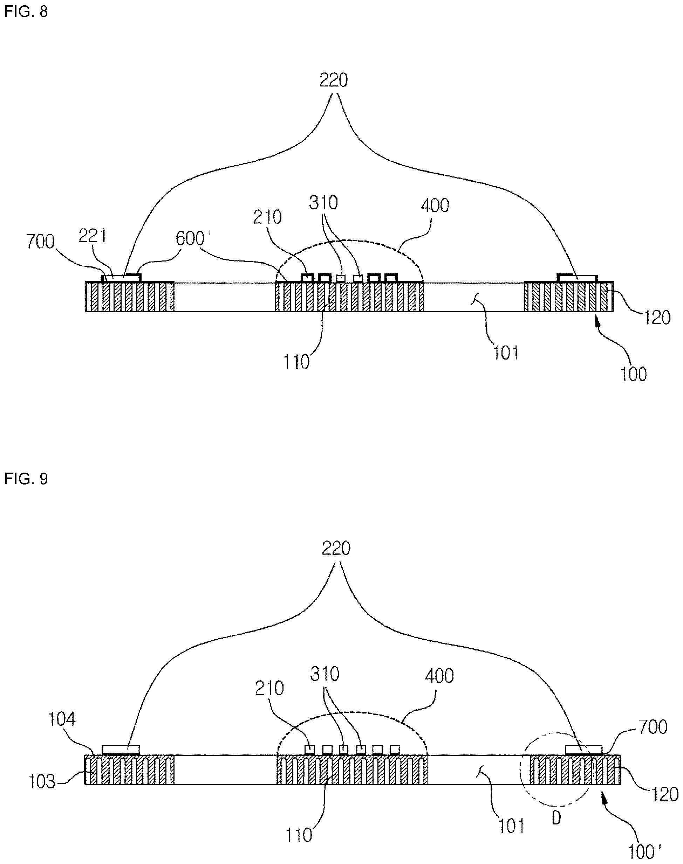

As illustrated in FIG. 8, the micro sensor provided with the micro heater according to the third embodiment includes a protective layer 600' formed only in a portion of the heater electrode or the sensor electrode. Thus, a non-protective portion 221 not covered with the protective layer is formed in the heater electrode or the sensor electrode.

The protective layer 600' is not formed in the regions of the sensor wiring lines 310 of the sensor electrode covered with the sensing material 400. Thus, the upper surfaces and the side surfaces of the sensor wiring lines 310 make direct contact with the sensing material 400. This enables a signal to be transmitted smoothly.

Furthermore, the protective layer 600' is not formed in some portions of the heater electrode pads 220 of the heater electrode and the sensor electrode pads of the sensor electrode. Thus, the protective layer 600' is formed in the heater wiring line 210, a portion of the heater electrode pads 220, a connection portion of the heater electrode pads 220 and the heater wiring line 210, a portion of the sensor electrode pads, and a connection portion of the sensor electrode pads and the sensor wiring lines 310. In other words, the non-protective portion 221 is formed in the region of the sensor electrode covered with the sensing material 400 and in a portion of the sensor electrode pads and the heater electrode pads 220.

More specifically, the protective layer 600' is not formed in the regions of the sensor electrode pads and the heater electrode pads 220, which adjoin the respective corners of the substrate 100. This makes it possible to smoothly perform soldering in the exposed regions.

Furthermore, the protective layer 600' is formed so as to cover the upper portions and the side portions of the heater electrode and the sensor electrode. The protective layer 600' is also formed on the substrate 100.

The protective layer 600' is formed in the first support portion 110 of the substrate 100 covered with the sensing material 400. However, the protective layer 600' may not be formed on the substrate 100 around the sensor wiring lines 310.

The protective layer 600' is formed in the upper portion of the second support portion 120 of the substrate 100 which supports the sensor electrode pads and the heater electrode pads 220. In the region of the substrate 100 where the protective layer 600' is formed, the top ends of the pores are closed by the protective layer 600'. The protective layer 600' is not formed in the regions of the substrate 100 where the air gaps 101 are formed.

### <Fourth Embodiment of the Present Invention>

Next, descriptions will be made on a micro sensor provided with a micro heater according to a fourth embodiment of the present invention. In the following descriptions, the components identical with or similar to those of the micro sensor and the micro heater of the first, second and third embodiments will be designated by like reference numerals with the detailed description and illustration thereof omitted.

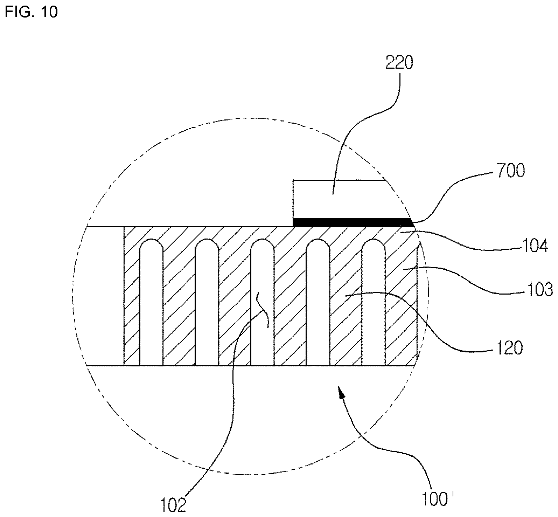

As illustrated in FIGS. 9 and 10, the micro sensor provided with the micro heater according to the fourth embodiment includes a substrate 100' formed of a porous layer 103 having a plurality of vertically-extending pores 102 and a barrier layer 104 positioned on the porous layer 103, a sensor electrode formed on the substrate 100', and a heater electrode formed on the substrate 100'. At least one of the sensor electrode and the heater electrode is formed on the barrier layer 104.

The substrate 100' includes a porous layer 103 and a barrier layer 104 formed on the porous layer 103. That is to say, the substrate 100' is formed of a porous material having a plurality of vertically-extending pores 102. For the sake of convenience in description, the diameter and length of the pores 102 are slightly exaggerated in the drawings.

The substrate 100' may be formed by anodizing an aluminum plate. Thus, the porous layer is a porous anodic aluminum oxide (AAO) layer. Aluminum is removed from the anodized aluminum substrate 100' so that only the porous layer 103 and the barrier layer 104 are left in the substrate 100'. The top ends of the pores 102 are closed by the barrier layer and the bottom ends of the pores 102 are opened. This enhances the heat insulation effect.

Unlike the foregoing descriptions, the barrier layer may be partially removed so that the pores of the porous layer can vertically extend through the substrate 100'. In other words, the barrier layer may be formed only in a portion of the porous layer. The porous layer may include pores vertically extending through the substrate 100'. In other words, only the porous layer may be left by removing the aluminum material and the barrier layer from the regions of the substrate other than the heater electrode and the sensor electrode.

The sensor electrode includes a first sensor electrode and a second sensor electrode spaced apart from the first sensor electrode. Each of the first and second sensor electrodes includes sensor wiring lines 310 and sensor electrode pads connected to the sensor wiring lines 310. The sensor electrode is formed on the upper surface of the barrier layer 104 of the substrate 100' from which the aluminum material is removed. The sensor electrode may be made of platinum (Pt).

The heater electrode includes a heater wiring line 210 disposed closer to the sensor wiring lines 310 than the sensor electrode pads, and heater electrode pads 220 connected to the heater wiring line 210. The heater electrode is formed on the upper surface of the barrier layer 104 of the substrate 100' from which the aluminum material is removed.

The sensor electrode and the heater electrode disposed on the barrier layer 104 are formed using a liquid photoresist (LPR). Due to the existence of the barrier layer 104, the liquid photoresist does not flow into the pores 102. Thus, the patterns of the sensor electrode and the heater electrode can be smoothly formed. It is also possible to accurately form the patterns of the sensor electrode and the heater electrode in a small size. The heater electrode and the sensor electrode may be formed by sputtering or the like.

If the porous layer 103 is supported by the barrier layer 104 when forming the heater electrode and the sensor electrode in this way, the porous layer 103 can be maintained even if the heater electrode and the sensor electrode are formed by sputtering or the like.

For example, the micro sensor may be manufactured by the following method. An aluminum substrate is subjected to an anodizing process. Thereafter, the aluminum is etched away. Then, a liquid photo resist is coated. Subsequently, a heater electrode and a sensor electrode are formed by sputtering. After the liquid photoresist is removed, air gaps 101 are formed by etching the substrate. The heater electrode is made of platinum (Pt).

The heater electrode includes a heater wiring line 210 disposed closer to the sensor wiring lines 310 than the sensor electrode pads, and heater electrode pads 220 connected to the heater wiring line 210.

A protective layer (not shown) made of tantalum oxide (TaOx) may be formed over the entire upper portions of the heater electrode and the sensor electrode. The protective layer prevents the heater electrode and the sensor electrode from being oxidized. Thus, the heater electrode and the sensor electrode can be protected by the protective layer.

Furthermore, a tantalum oxide layer 700 is disposed between the barrier layer 104 of the substrate 100' and the heater electrode and the sensor electrode. The tantalum oxide layer 700 enhances the bonding strength of the heater electrode and the sensor electrode.

Due to the existence of the air gaps 101, a first support portion 110 which supports both the heater wiring line 210 and the sensor wiring lines 310 and a second support portion 120 which supports the heater electrode pads 220 and the sensor electrode pads are formed in the substrate 100'. A sensing material 400 is formed so as to cover the upper surfaces and the side surfaces of the heater wiring line 210 and the sensor wiring lines 310 and so as to cover the upper surface of the first support portion 110.

While preferred embodiments of the present invention have been described above, a person skilled in the relevant technical field will be able to differently change or modify the present invention without departing from the spirit and scope of the present invention defined in the claims.

## Claims

1. A micro heater, comprising:
a substrate; and
a heater electrode formed on the substrate,
wherein a protective layer is formed on the heater electrode.

2. The micro heater of claim 1, wherein the heater electrode is made of at least one member selected from a group consisting of Pt, W, Co, Ni, Au, Cu and C and a mixture thereof, and
the protective layer is made of at least one member selected from a group consisting of tantalum oxide, titanium oxide, silicon oxide, aluminum oxide, indium tin oxide, indium oxide and tin oxide.

3. The micro heater of claim 2, wherein the heater electrode is made of at least one member selected from a group consisting of Pt, W, Co, Ni, Au, Cu and C and a mixture thereof,
an intermediate layer is disposed between the substrate and the heater electrode, and
the intermediate layer is made of at least one member selected from a group consisting of tantalum oxide, titanium oxide, silicon oxide, aluminum oxide, indium tin oxide, indium oxide and tin oxide.

4. The micro heater of claim 2, wherein the protective layer is formed in a portion of the heater electrode, and
a non-protective portion in which the protective layer is not formed is formed in the heater electrode.

5. The micro heater of claim 4, wherein the heater electrode includes a heater wiring line and heater electrode pads connected to the heater wiring line, and
the non-protective portion is formed on a portion of the heater electrode pads.

6. A micro sensor, comprising:
a substrate;
a sensor electrode formed on the substrate; and
a heater electrode formed on the substrate,
wherein a protective layer is formed on an upper portion of at least one of the heater electrode and the sensor electrode.
